# EUROPEAN PATENT APPLICATION

(11) **EP 2 128 123 A1**
(43) Date of publication of application: **02.12.2009**
(21) Application number: 08704108.3
(22) Date of filing: 29.01.2008
(51) Int. Cl.: C07C 229/42, A61K 31/192, A61K 31/196, A61K 31/405, A61K 31/407, A61P 29/00, A61P 43/00, C07C 57/58, C07C 59/84, C07C 211/09, C07C 215/08, C07C 217/10, C07C 217/62, C07C 237/04, C07D 209/28, C07D 491/052

(54) **SALT OF NONSTEROIDAL ANTI-INFLAMMATORY DRUG AND ORGANIC AMINE COMPOUND AND USE THEREOF**

(30) Priority: 29.01.2007 JP 2007018005; 09.02.2007 JP 2007031188; 16.03.2007 JP 2007069620
(71) Applicant: Medrx Co., Ltd., Higashikagawa-shi Kagawa 769-2712 (JP)
(72) Inventor: HANMA, Noritaka, Higashikagawa-shi Kagawa 769-2712 (JP); MIWA, Yasushi, Higashikagawa-shi Kagawa 769-2712 (JP); HAMAMOTO, Hidetoshi, Higashikagawa-shi Kagawa 769-2712 (JP)
(74) Representative: Duckworth, Timothy John
(86) International application number: PCT/JP2008/051326
(87) International publication number: WO 2008/093686

(57) **Abstract**

The present invention provides a salt, particularly an ionic liquid, of a non-steroidal anti-inflammatory drug (NSAID) having carboxylic acid and an organic amine compound, which has the properties necessary for use as an external preparation (free of crystal precipitation, stable, easily soluble in organic solvents and the like often used for external preparations, and the like).

The solving means is a 1:1 salt or ionic liquid of a carboxylic acid NSAID and an organic amine compound.

## Description

### Technical Field

The present invention relates to a novel 1:1 salt of a non-steroidal anti-inflammatory drug (hereinafter sometimes to be abbreviated as NSAID) having carboxylic acid and an organic amine compound, and use thereof as an external preparation. Particularly, the present invention relates to a novel Brönsted type ionic liquid of NSAID and use thereof.

### Background Art

While non-steroidal anti-inflammatory drugs (NSAID) are widely used for various inflammatory diseases, the risk of digestive tract disorder, which is a side effect of NSAID, has been considered a problem as the frequency of use thereof increases. The risk is considered to be attributable to the gastrointestinal mucosa disorder property of NSAID itself, and its action to delay cure of ulcer.
Since NSAID is often used as an oral preparation, the risk of digestive tract disorder increases further.

To avoid the risk of digestive tract disorder, parenteral administration methods have been considered, and particularly, a method for transdermal absorption of NSAID in the form of an external preparation has been tried. For example, a patch of indomethacin is a typical successful case.
Furthermore, as use of NSAID as external preparations, ionic liquids of a 1:1 salt of indomethacin etc. and lidocain are disclosed and reported to improve skin permeability (patent document 1). In addition, an ionic liquid of a salt of etodolac and lidocain, which are poorly stable to light, is disclosed, and possible production of an external preparation having photostability is shown (patent document 2). Furthermore, an equimolar salt of a non-steroidal anti-inflammatory drug (NSAID) and a topical anesthetic is disclosed (patent document 3). In Examples, however, only two Production Examples of diclofenac-lidocain salt and ketorolaclidocain salt are disclosed.

On the other hand, a salt formed from diclofenac and tramadol is disclosed, though it is not an external preparation, and the salt is shown to be suitable for use as a sustained release oral preparation of tramadol (patent document 4).

As mentioned above, salts of some compounds of NSAIDs and lidocain, and use thereof as external preparations are known. However, conversion of other NSAID compounds to ionic liquids and use thereof as external preparations are not known at all. Furthermore, properties of ionic liquids relating to NSAID compounds are not much known. For example, an ionic liquid of indomethacin and lidocain (1:1 salt) was a highly viscose ionic liquid immediately after synthesis, but crystals were gradually precipitated when stood still at room temperature (mp. 116 - 117°C) (patent document 1). The crystals were those of a salt formed from indomethacin (2 mol) and lidocain (1 mol). Since the indomethacin:lidocain 2:1 crystals are thermodynamically stable, the equilibrium is considered to have gradually shifted from the ionic liquid (1:1 salt) to the crystal.

Thus, not all the lidocain salts of NSAID become ionic liquids, and whether or not they are thermodynamically stable can be known only after they are concretely prepared and examined, even when prepared as 1:1 salts.

As for the transdermal absorption action of NSAID, particularly good results have not been obtained in the previous reports. For example, loxoprofen as a salt of sodium loxoprofen is widely used as an antiphlogistic analgetic for chronic rheumatoid arthritis, osteoarthritis and the like, and further as an antipyretic analgesic for acute upper respiratory inflammation. Although a patch of the sodium salt is commercially available, it shows poor transdermal absorbability, and the blood concentration of its active metabolite (trans-OH form) remains at 10 - 30 ng/mL. Therefore, the metabolite has been reported to show an inferior effect as compared to oral preparations, even though systemic side effects are absent. As for external preparations of loxoprofen sodium, therefore, it is considered that a very good effect cannot be expected for strong inflammations of major joints, particularly knees and the like. Therefore, improvement of transdermal absorbability of loxoprofen sodium salt to increase applicability as an antiphlogistic for major joints such as knees and the like is expected. However, a drastic improvement has not been observed.

For example, for use of an anti-inflammatory drug (having a carboxyl group in a molecule) containing loxoprofen and the like as an external preparation, l-menthol is used to promote absorption of the main drug. However, a long-term preservation of a preparation containing l-menthol results in a reaction between a carboxyl group and 1-menthol to form an l-menthol ester form, whereby the amount of the main drug decreases and the like. Thus, a metal hydroxide is used (patent document 3), or a fatty acid metal salt is used (patent document 4). It is shown, however, that the stability of the drug improves in both cases, but the transdermal absorbability of the drug itself is not markedly improved.

On the other hand, improvement of transdermal absorbability using a medicament having a carboxyl group and an ionic liquid forming a counter ion is shown, but the effect and usefulness of a 1:1 salt (mainly ionic liquid) as an external preparation are not sufficiently disclosed (patent document 5). For example, an equimolar salt of a non-steroidal anti-inflammatory drug (NSAID) and a topical anesthetic is disclosed, which can also be used as an external preparation and can improve transdermal absorbability. However, an effect thereof is not shown (patent document 5). Moreover, a diclofenac tramadol salt used as an oral preparation is disclosed (patent document 6). However, usefulness as external preparation is not shown at all.

patent document 1: JP-A-2005-82512
patent document 2: WO 2006-018987
patent document 3: JP-A-2005-314328
patent document 4: JP-B-3238409
patent document 5: JP-A-2004-285044
patent document 6: JP-A-2004-514692

### Disclosure of the Invention

### Problems to be Solved by the Invention

The present invention aims to provide a novel 1:1 salt compound of a carboxylic acid NSAID and an organic amine compound. Particularly, it aims to provide a stable ionic liquid of NSAID. Furthermore, it aims to provide an ionic liquid having high external preparation characteristics, which shows high solubility in a solvent and high transdermal absorbability.

### Means of Solving the Problems

The present inventors have conducted intensive studies in an attempt to solve the aforementioned problems. As a result, they have found that once a salt is formed, infrared absorption spectrum of carboxylic acid disappears, a new infrared absorption spectrum of carboxy ion is developed, and the infrared absorption spectrum can be used for the judgment of the presence or absence of salt formation, based on which they have found a salt of NSAID and an organic amine compound (produced in equimolar), which is superior in solubility and transdermal absorbability, and stable as an ionic liquid at ambient temperature, which resulted in the completion of the present invention.

Accordingly, the present invention provides the following.
[1] A 1:1 salt comprising a carboxylic acid non-steroidal anti-inflammatory drug below and an organic amine compound,
   a) salts of indomethacin:
      dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
   b) salts of ketoprofen:
      lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
   c) salts of flurbiprofen:
      lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
   d) salts of diclofenac:
      dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
   e) salts of etodolac:
      lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
   f) salts of loxoprofen:
      lidocain salt, dibucaine salt, bupivacaine salt,
      diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt.
[2] An ionic liquid which is a 1:1 salt comprising a carboxylic acid non-steroidal anti-inflammatory drug below and an organic amine compound, and is a liquid at ambient temperature (25°C),
   a) salts of indomethacin:
      dibucaine salt, bupivacaine salt, diphenhydramine salt, tramadol salt, eperisone salt, tolperisone salt, dextromethorphan salt, donepezil salt, triethanolamine salt, triisopropanolamine salt, 3-dimethylamino-1-propylamine salt,
   b) salts of ketoprofen:
      lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
   c) salts of flurbiprofen:
      lidocain salt, dibucaine salt, bupivacaine salt, diphenhydramine salt, tramadol salt, eperisone salt, tolperisone salt, dextromethorphan salt, donepezil salt, diethanolamine salt, diisopropanolamine salt, triisopropanolamine salt, 3-dimethylamino-1-propylamine salt,
   d) salts of diclofenac:
      lidocain salt, bupivacaine salt, diphenhydramine salt, eperisone salt, tolperisone salt, dextromethorphan salt, donepezil salt,
   e) salts of etodolac:
      lidocain salt, dibucaine salt, bupivacaine salt, diphenhydramine salt, eperisone salt, tolperisone salt, dextromethorphan salt, diethanolamine salt, diisopropanolamine salt, triisopropanolamine salt, donepezil salt, 3-dimethylamino-1-propylamine salt,
   f) salts of loxoprofen:
      lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt.
[3] An external preparation comprising the ionic liquid of the above-mentioned [2].
[4] A method of converting a carboxylic acid NSAID to an ionic liquid, which comprises adding an organic amine compound to a carboxylic acid NSAID, wherein the organic amine compound is at least one kind selected from the group consisting of lidocain, dibucaine, bupivacaine, diphenhydramine, tramadol, eperisone, tolperisone, dextromethorphan, donepezil, diethanolamine, triethanolamine, diisopropanolamine, triisopropanolamine and 3-dimethylamino-1-propylamine.
[5] The method of the above-mentioned [4], wherein the carboxylic acid NSAID and the organic amine compound form an equimolar salt.
[6] The method of the above-mentioned [4] or [5], wherein the carboxylic acid NSAID is selected from the group consisting of indomethacin, ketoprofen, flurbiprofen, etodolac and loxoprofen.
[7] The method of any one of the above-mentioned [4] to [6], wherein the carboxylic acid NSAID is loxoprofen. Effect of the Invention

The novel 1:1 equimolar salt compound (mainly ionic liquid) of a carboxylic acid NSAID and an organic amine compound of the present invention is stable at ambient temperature, and shows an improved solubility in a liposoluble solvent. Therefore, it can increase the content of the carboxylic acid NSAID in an external preparation such as a patch and the like. As the result, transdermal absorbability and skin permeability of the efficacy ingredient can be enhanced. Therefore, the ionic liquid of the present invention can be effectively used as an external preparation.

### Brief Description of the Drawings

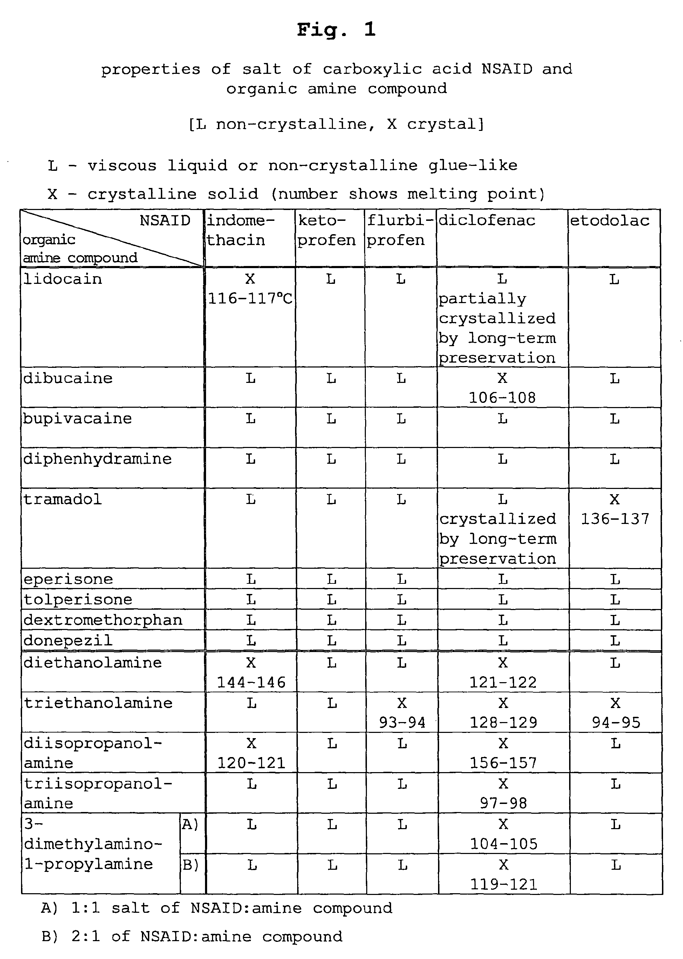

Fig. 1 shows the form of a salt of a carboxylic acid NSAID and an organic amine compound, wherein L shows a liquid form at ambient temperature (25°C), and X shows a crystalline salt. The crystalline salt was isolated, and the melting point was measured, which is described in the column thereof. Best Mode for Carrying out the Invention

The present invention provides a 1:1 salt of a carboxylic acid non-steroidal anti-inflammatory drug (NSAID) and an organic amine compound.

The carboxylic acid NSAID (sometimes to be referred to as the NSAID of the present invention) in the present invention is not particularly limited as long as it contains carboxylic acid in the structure thereof.

Examples of the NSAID of the present invention include indomethacin, ketoprofen, flurbiprofen, diclofenac, etodolac, loxoprofen, ibuprofen, naproxen, mefenamic acid, floctafenine, sulindac, fenbufen, mofezolac and the like, with preference given to indomethacin, ketoprofen, flurbiprofen, diclofenac, etodolac and loxoprofen.

The organic amine compound in the present invention means a basic compound having a primary, secondary or tertiary amine residue, which can form a salt by reacting with an acidic NSAID.

Examples of the organic amine compound in the present invention include lidocaine, dibucaine, bupivacaine, diphenhydramine, tramadol, eperisone, tolperisone, dextromethorphan, donepezil, diethanolamine, triethanolamine, diisopropanolamine, triisopropanolamine, 3-dimethylamino-1-propylamine, procaine, mepivacaine, oxybutynin, fentanyl, pergolide, tamsulosin, risperidone, flurazepam, butorphanol, perisoxal, pridinol, trihexyphenidyl, amantadine, tulobuterol, isoprenaline, propranolol, ketotifen, difenidol, morphine, meloxicam, valdecoxib, celecoxib and the like.

The 1:1 salt of a carboxylic acid NSAID and an organic amine compound in the present invention means a Brönsted type salt obtained by reaction of 1:1 equimolar amounts of the former and the later (hereinafter sometimes to be referred to as the salt of the present invention).

Examples of the salt of the present invention include the salts of the following a) - f).
a) salts of indomethacin:
   dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
b) salts of ketoprofen:
   lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
c) salts of flurbiprofen:
   lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
d) salts of diclofenac:
   dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
e) salts of etodolac:
   lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
f) salts of loxoprofen:
   lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt.

The salt of the present invention can be produced by reacting equimolar amounts of known Brönsted type acid and a base. For example, equimolar amounts of an NSAID and an organic amine compound are dissolved in an organic solvent, and the precipitated salt is collected, or the organic solvent is evaporated to give the corresponding salt.

The "ionic liquid" of the present invention is a salt obtained by reacting equimolar amounts of an NSAID having carboxylic acid and an organic amine compound, which is a liquid at ambient temperature (25°C). The liquid is generally non-crystalline and viscous. The viscous liquid means a starch syrup-like state, molten glue, and the like.

Examples of the ionic liquid of the present invention include salts of the following a) - f).
a) salts of indomethacin:
   dibucaine salt, bupivacaine salt, diphenhydramine salt, tramadol salt, eperisone salt, tolperisone salt, dextromethorphan salt, donepezil salt, triethanolamine salt, triisopropanolamine salt, 3-dimethylamino-1-propylamine salt,
b) salts of ketoprofen:
   lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
c) salts of flurbiprofen:
   lidocain salt, dibucaine salt, bupivacaine salt, diphenhydramine salt, tramadol salt, eperisone salt, tolperisone salt, dextromethorphan salt, donepezil salt, diethanolamine salt, diisopropanolamine salt, triisopropanolamine salt, 3-dimethylamino-1-propylamine salt,
d) salts of diclofenac:
   lidocain salt, bupivacaine salt, diphenhydramine salt, eperisone salt, tolperisone salt, dextromethorphan salt, donepezil salt,
e) salts of etodolac:
   lidocain salt, dibucaine salt, bupivacaine salt, diphenhydramine salt, eperisone salt, tolperisone salt, dextromethorphan salt, diethanolamine salt, diisopropanolamine salt, triisopropanolamine salt, donepezil salt, 3-dimethylamino-1-propylamine salt,
f) salts of loxoprofen:
   lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt.

Examples of the production method of the ionic liquid of the present invention include a method comprising dissolving equimolar amounts of a carboxylic acid NSAID and an organic amine compound in an organic solvent such as methanol, chloroform and the like, and then evaporating the solvent under reduced pressure to give a viscous Brönsted type ionic liquid.

The external preparation of the present invention contains the above-mentioned ionic liquid.
In the external preparation of the present invention, the concentration of the above-mentioned ionic liquid varies depending on the kind and dosage form of the carboxylic acid NSAID and the organic amine compound contained therein. Generally, 2 - 40 wt%, preferably 2 - 20 wt%, of the above-mentioned ionic liquid is contained.
The dosage form of the external preparation of the present invention is not particularly limited as long as it can be topically administered to the skin. Examples thereof include ointment, cream, gel, cataplasm, tape, lotion, aerosol, plaster, patch, liquid, liniment and the like. Among these, ointment, plaster, cream, gel, cataplasm and tape are preferable.

The external preparation of the present invention can contain, according to the dosage form thereof, known additives generally used by those of ordinary skill in the art. Examples of such additive include base, fatty acid or a derivative thereof, alcohol, surfactant, suspending agent, thickener, solvent, solubilizing agent, inorganic particle, excipient, stabilizer, wetting agent, buffering agent, pH adjuster, colorant, flavor, binder, propellant and the like.

Examples of the above-mentioned base include components of oily base or hydrophobic base, components of hydrophilic base or water-separation base, gel base and the like. Examples of the above-mentioned components of oily base or hydrophobic base include rubbers such as natural rubber, isoprene rubber, polyisobutylene, styrene-isoprene-styrene block copolymer, styrene-butadiene-styrene block copolymer, styrene-ethylenebutylene-styrene block copolymer, (meth)acrylic acid alkylester (co)polymer, polyacrylate, methacrylate, polybutene, liquid polyisoprene and the like, adhesives such as polystyrene resin, aromatic petroleum resin, hydrocarbon gel, aliphatic saturated hydrocarbon resin, hydrogenated petroleum resin and the like, softening agents such as petrolatum, cetanol, beeswax, white beeswax, lanolin, purified lanolin, liquid paraffin, paraffin wax, plastibase containing liquid paraffin and polyethylene, silicone oil, triglyceride, squalene, microcrystalline wax, whale wax, etc. and the like.

Examples of the above-mentioned components of hydrophilic base or water-separation base include hydrophilic fatty acid ester such as glycerol ester of saturated fatty acid and the like, water-soluble polymer such as polyethylene glycol, etc. and the like.
Examples of the above-mentioned component of gel base include cellulose derivative such as carboxyvinyl polymer, acrylic acid starch, sodium polyacrylate, tragacanth, alginates, methylcellulose, carmellose, carmellose sodium, hydroxypropylcellulose, hydroxypropylmethylcellulose and the like, colloidal clay comprising silicic acid salt such as bentonite, veegum and the like, water-soluble basic substance such as carboxyvinyl polymer, polyvinyl alcohol, polyvinylpyrrolidone, hydroxide alkali, alkanol amine, etc. and the like.

Examples of the above-mentioned fatty acid or a derivative thereof include higher fatty acid such as oleic acid, stearic acid and the like or a salt thereof, ester with higher fatty acid and monovalent aliphatic alcohol such as caprylic acid, caproic acid, myristic acid, palmitic acid, stearic acid, oleic acid and the like (e.g., isopropyl myristate, isopropyl palmitate, isopropyl stearate, decyl oleate etc.), triglyceride such as caprylic acid triglyceride, caproic acid triglyceride, peanuts oil, castor oil, cacao oil, hydrogenated fats and oils (e.g., hydrogenated castor oil etc.) and the like, fatty acid ester of polyvalent alcohol such as pentaerythritol fatty acid ester, etc. and the like. Examples of the ester of polyvalent carboxylic acid and alcohol include ester of polyvalent carboxylic acid such as adipic acid, sebacic acid and the like, and monovalent aliphatic alcohol (e.g., ethyl adipate, diisopropyl adipate etc.) and the like.

Examples of the above-mentioned alcohol include higher alcohol such as benzyl alcohol, lauryl alcohol, myristyl alcohol, cetyl alcohol, stearyl alcohol, cetostearyl alcohol, 2-octyldodecanol and the like, lower alcohol such as ethanol, isopropanol and the like, or polyvalent alcohol such as ethylene glycol, glycerol, propylene glycol, 1,3-butylenealcohol, etc. and the like.

Examples of the above-mentioned surfactant include natural emulsifier such as gum arabic, gelatin, tragacanth, lecithin, cholesterol and the like, anionic surfactant such as soap, sodium alkyl sulfate and the like, sorbitan polyoxyethylene fatty acid ester such as monooleyl sorbitan polyoxyethylene and the like, glycerol fatty acid ester such as polyoxyethylene castor oil derivative, polyoxyethylene hydrogenated castor oil, glycerol monostearate, sorbitan monooleate and the like, sorbitan fatty acid ester such as sorbitan monostearate, sorbitan sesquioleate and the like, polyoxyethylene higher alcohol ether such as polyoxyethylene cetyl ether and the like, nonionic surfactant such as polyoxyethylene alkylphenol, polyoxyethylene oxypropylene copolymer (e.g., pluronic etc.) and the like, cationic surfactant such as cetyltrimethylammonium chloride and the like, ampholytic surfactant and the like.

Examples of the above-mentioned suspending agent or thickener include polysaccharides such as gum arabic, tragacanth, pullulan, locust bean gum, tamarind gum, pectin, xanthan gum, guar gum, carageenan and the like, methylcellulose, carmellose, carmellose sodium, polyvinyl alcohol, polyvinylpyrrolidone, acrylic acid copolymer, carboxyvinyl polymer, colloidal microcrystalline cellulose and the like.

Examples of the above-mentioned solvent include water, propylene glycol, butylene glycol, isopropanol, ethanol, glycerol, diethyl sebacate, isopropyl myristate, diisopropyl adipate, myristyl palmitate, stearyl stearate, myristyl myristate, seril lignocerate, lacceril cerolate, lacceril laccerate and the like.

Examples of the above-mentioned solubilizing agents include carmellose sodium, propylene glycol, polysorbate 80, sodium benzoate, benzyl benzoate, urethane, monoethanolamine, diethanolamine, glycerol, sodium salicylate, diethylacetamide, sodium hydroxide, sodium carbonate, urea, N-hydroxyethyllactamide, monomethylacetamide, lidocain and the like.

Examples of the above-mentioned inorganic particle include talc, silicic anhydride, calcium carbonate, magnesium carbonate, colloidal silica, bentonite and the like.

Examples of the above-mentioned excipient include saccharides such as sucrose and the like, sugar alcohol such as mannitol and the like, starch derivative such as dextrin and the like, cellulose derivative such as crystalline cellulose and the like, inorganic substance such as calcium phosphate, etc. and the like.

Examples of the above-mentioned stabilizer include preservative, antioxidant and the like. Examples of the above-mentioned preservative include parahydroxybenzoic acid esters such as methylparaben, propylparaben and the like, alcohols such as chlorobutanol, benzyl alcohol, phenylethyl alcohol and the like, thimerosal, acetic anhydride, sorbic acid and the like. Examples of the above-mentioned antioxidant include sodium bisulfite, L-ascorbic acid, sodium ascorbate, butylhydroxyanisole, butylhydroxytoluene, propyl gallate, tocopherol acetate, dl-α-tocopherol and the like.

Examples of the above-mentioned wetting agent include polyvalent alcohol such as glycerol, propylene glycol, butylene glycol, sorbitol, etc. and the like.

In addition to these additives, the above-mentioned buffering agent, pH adjuster, colorant, flavor, binder, propellant and the like can also be added as necessary. Furthermore, for example, peppermint oil, 1-menthol, camphor, thymol, tocopherol acetate, glycyrrhizinic acid, nonanoic acid vanillylamide, chili pepper extract and the like can also be added.

Besides these additives, as long as the action and effect of the external preparation of the present invention is not prevented, a pharmaceutical product containing other drugs can also be added.

The additives exemplified above are appropriately selected according to the dosage form of the external preparation of the present invention, and the amount thereof to be added is also appropriately selected within the range generally employed according to each dosage form.

Now, the production method of the external preparation of the present invention is explained. The external preparation of the present invention can be produced by a suitable method known to those of ordinary skill in the art, and the production method is not particularly limited. For example, the external preparation of the present invention can be obtained by adding the ionic liquid of the present invention to, for example, a suitable base shown above, which is known to those of ordinary skill in the art, and further adding other suitable additives shown above and mixing them. Where necessary, they can also be heated.

The external preparation of the present invention can be applied to an affected part according to the dosage form. The application frequency of the above-mentioned external preparation to the affected part can be determined according to the content of the main drug and the like. For example, the preparation can be applied once or more than once per day, where the application frequency is not particularly limited.
The dose of the external preparation of the present invention may be an effective amount of an NSAID contained in the external preparation. While the effective amount of NSAID varies depending on the kind, body weight of a subject, severity and the like, in the case of indomethacin, for example, it is generally 50 - 200 mg/day, preferably 72 - 144 mg/day, as indomethacin (free form) for an adult.

As another embodiment of the present invention, a method of converting NSAID to an ionic liquid, including adding an organic amine compound to NSAID is provided.
In addition, a method of converting NSAID to an ionic liquid, including forming an equimolar salt of NSAID and an organic amine compound is provided. The combination of NSAID and an organic amine compound is the same as that described for the above-mentioned ionic liquid of the present invention.
For example, it is a method of converting loxoprofen to an ionic liquid, including adding an organic amine compound to loxoprofen, wherein the organic amine compound is at least one kind selected from the group consisting of lidocain, dibucaine, bupivacaine, diphenhydramine, tramadol, eperisone, tolperisone, dextromethorphan, donepezil, diethanolamine, triethanolamine, diisopropanolamine, triisopropanolamine and 3-dimethylamino-1-propylamine. The amounts of loxoprofen and an organic amine compound to be added for conversion to an ionic liquid in a molar ratio is 0.7 - 1.5 of the organic amine compound relative to loxoprofen as 1. A preferable molar ratio is equimol. According, it is a method of converting loxoprofen to an ionic liquid, comprising forming an equimolar salt of loxoprofen and an organic amine compound.
Similarly, other NSAIDs other than loxoprofen can be converted to an ionic liquid by adding an organic amine compound in a molar ratio of 0.7 - 1.5. A preferable molar ratio is equimol. According, it is a method of converting NSAID to an ionic liquid, comprising forming an equimolar salt of NSAID and an organic amine compound.

The present invention is explained in more detail in the following by referring to Examples. The present invention is not limited to the following Examples but can be performed upon appropriate modification, both of which are encompassed in the technical scope of the present invention.

### Examples

### Measurement method of salt formation

First, a salt was produced using equimolar amounts of a carboxylic acid NSAID and organic amine. As the NSAID, indomethacin, ketoprofen, flurbiprofen, diclofenac, etodolac and loxoprofen were used, and as the organic amine compound, lidocain, dibucaine, bupivacaine, diphenhydramine, tramadol, eperisone, tolperisone, dextromethorphan, donepezil, diethanolamine, triethanolamine, diisopropanolamine, triisopropanolamine and 3-dimethylamino-1-propylamine were used. The obtained ionic liquid or crystals and the like were left standing, confirmed to be stable, and then the forms thereof were observed. The results are shown in Fig. 1. This has clarified that the salt produced by the combination of NSAID and an organic amine compound may be a liquid (viscous liquid) or crystal at ambient temperature (25°C).

Then, an ionic liquid was dissolved in chloroform or applied to a rock salt plate, and infrared absorption spectrum was measured using an infrared spectrophotometer (FTIR-8400S) manufactured by SHIMADZU Corporation. The absorption spectrum of carboxylic acid of the above-mentioned NSAID (starting material) was evaluated. As a result, it was observed that the infrared absorption spectrum of carboxylic acid disappeared when a salt was produced, and an absorption spectrum of carboxy ion was newly developed. Hence, it was clarified that the presence or absence of salt formation can be evaluated based on the infrared absorption spectrum.

### (Example 1) Synthesis of salt (ionic liquid) of indomethacin and an organic amine compound

### (1) Synthesis of indomethacin-eperisone salt

### a) When eperisone is used as an organic amine compound:

Indomethacin (3.58 g, 10 mmol) and eperisone (2.59 mg, 10 mmol) were dissolved in methanol (20 mL), and methanol was evaporated under reduced pressure to give an indomethacin-eperisone salt as a yellow starch syrup-like viscous liquid.
infrared absorption spectrum (chloroform): 1680 cm-1 (amidecarbonyl of indomethacin and carbonyl of eperisone), 1595 cm-1 (-COO⁻, -COOH, 1715 cm-1 by Nujol)
In the infrared absorption spectrum, the absorption spectrum (1715 cm-1) of carboxylic acid of indomethacin (starting material) disappeared, and an absorption spectrum (1595 cm-1) of carboxyl ion appeared newly. Thus, since it was found that disappearance of the starting material and production of a salt can be confirmed using an infrared absorption spectrum, the presence or absence of production of Brönsted type ionic liquid was confirmed using an infrared absorption spectrum in the following Examples.

### b) When eperisone hydrochloride is used as an organic amine compound:

Indomethacin (358 mg, 1 mmol) and eperisone hydrochloride (395 mg, 1 mmol) were dissolved in methanol (3 mL), and 1N aqueous sodium hydroxide solution (1 mL) was added. The solution was concentrated under reduced pressure, 2-propanol (10 mL) was added, and the mixture was again concentrated under reduced pressure. 2-Propanol (10 mL) was added to the residue, and the resulting precipitate was filtered off. The filtrate was concentrated under reduced pressure to give an indomethacin-eperisone salt as a yellow starch syrup-like viscous liquid. This product showed an infrared absorption spectrum similar to the above-mentioned a).

### (2) Synthesis of indomethacin-diphenhydramine salt

Indomethacin (358 mg, 1 mmol) and diphenhydramine (255 mg, 1 mmol) were stirred at 50 - 60°C for 30 min to give an indomethacin-diphenhydramine salt as a yellow starch syrup-like viscous liquid.
infrared absorption spectrum (liquid membrane): 1680 cm-1 (amidecarbonyl of indomethacin), 1595 cm-1 (COO-)

### (3) Synthesis of other indomethacin salts

In the same manner as in the above, indomethacin salts shown in Table 1 were produced as follows. The absorption spectrum of carboxylic acid of indomethacin (starting material) disappeared completely, and new absorption spectra of carboxyl ion shown in the following Table 1 appeared.

**[Table 1]**

| sample No. | organic amine compound | indomethacin salt IR absorption (-COOH) CM⁻¹ | form/note |
|---|---|---|---|
| | none | 1715 (Nujol) | |
| 1 | tolperisone | 1605 (chloroform) | viscous liquid |
| 2 | tramadol | 1595 (chloroform) | viscous liquid |
| 3 | dextromethorphan | 1600 (chloroform) | glue-like |
| 4 | dibucaine | 1595 (chloroform) | viscous liquid |
| 5 | lidocain | 1595 (Nujol) | indomethacin-lidocain 2:1 crystal mp 116 - 117°C |
| 6 | bupivacaine | 1590 (chloroform) | viscous liquid |
| 7 | donepezil | 1600 (chloroform) | glue-like |
| 8 | diethanolamine | 1580 (Nujol) | crystal mp 144 - 146°C |
| 9 | triethanolamine | 1580 (chloroform) | glue-like |
| 10 | diisopropanolamine | 1570 (Nujol) | crystal mp 120 - 121°C |
| 11 | triisopropanolamine | 1570 (chloroform) | viscous liquid |
| 12 | 3-dimethylamino-1-propylamine | 1580 (chloroform) 1580 (chloroform) | viscous liquid (1:1 salt) glue-like (1:2 salt) |

### (Example 2) Synthesis of salt (ionic liquid) of diclofenac and organic amine compound

### (1) Synthesis of diclofenac-eperisone salt

Diclofenac sodium (318 mg, 1 mmol) and eperisone hydrochlorate (296 mg, 1 mmol) were dissolved in methanol (5 mL) by heating. The solution was concentrated under reduced pressure, 2-propanol was added to the residue, and the resulting precipitate was filtered off. The filtrate was concentrated under reduced pressure to give a diclofenac-eperisone salt as a colorless glue-like product.
infrared absorption spectrum (chloroform): 1680 cm-1 (carbonyl of eperisone), 1605 cm-1 (COO-, COOH of diclofenac free form was 1965 cm-1)

### (2) Synthesis of other diclofenac salts

In the same manner as in the earlier sections or Example 1, diclofenac salts shown in the following Table 2 were produced. In addition, disappearance of the absorption spectrum of carboxylic acid (starting material) from an infrared absorption spectrum and appearance of new absorption spectrum of a carboxyl ion are shown in Table 2.

**[Table 2]**

| sample No. | organic amine compound | diclofenac salt IR absorption (-COOH) CM⁻¹ | form/note |
|---|---|---|---|
| | none | 1695 (Nujol) | |
| 1 | tramadol | 1590 (chloroform) | glue-like, crystallized during long-term preservation |
| 2 | dextromethorphan | 1610 (chloroform) | viscous liquid |
| 3 | diphenhydramine | 1595 (chloroform) | viscous liquid |
| 4 | dibucaine | 1590 (Nujol) | crystal mp 106 - 108°C |
| 5 | lidocain | 1600 (chloroform) | glue-like, partial crystallization during long-term preservation |
| 6 | bupivacaine | 1585 (chloroform) | glue-like |
| 7 | tolperisone | 1600 (chloroform) | viscous liquid |
| 8 | donepezil | 1605 (chloroform) | glue-like |
| 9 | diethanolamine | 1570 (Nujol) | crystal mp 121 - 122°C |
| 10 | triethanolamine | 1580 (Nujol) | crystal mp 128 - 129°C |
| 11 | diisopropanolamine | 1575 (Nujol) | crystal mp 156 - 157°C |
| 12 | triisopropanolamine | 1580 (Nujol) | crystal mp 97 - 98°C |
| 13 | 3-dimethylamino-1-propylamine | 1570 (Nujol) 1570 (Nujol) | mp 106 - 108°C (1:1 salt) mp 119 - 121°C (1:2 salt) |

### (Example 3) Synthesis of salt (ionic liquid) of ketoprofen and an organic amine compound

In the same manners as in Example 1 and Example 2, ketoprofen salts (ionic liquid) shown in the following Table 3 were produced. In addition, disappearance of the absorption spectrum of carboxylic acid (starting material) from an infrared absorption spectrum and appearance of new absorption spectrum of a carboxyl ion are shown in Table 3.

**[Table 3]**

| sample No. | organic amine compound | ketoprofen salt IR absorption (-COOH) CM⁻¹ | form/note |
|---|---|---|---|
| | none | 1700 (chloroform) | |
| 1 | eperisone | 1600 (neat) | viscous liquid |
| 2 | tolperisone | 1605 (neat) | viscous liquid |
| 2 | tramadol | 1600 (chloroform) | viscous liquid |
| 3 | dextromethorphan | 1605 (chloroform) | viscous liquid |
| 4 | diphenhydramine | 1595 (neat) | viscous liquid |
| 5 | dibucaine | 1595 (chloroform) | viscous liquid |
| 6 | lidocain | 1595 (chloroform) | viscous liquid |
| 7 | bupivacaine | 1585 (chloroform) | viscous liquid |
| 8 | donepezil | 1600 (chloroform) | viscous liquid |
| 9 | diethanolamine | 1575 (chloroform) | viscous liquid |
| 10 | triethanolamine | 1580 (chloroform) | viscous liquid |
| 11 | diisopropanolamine | 1580 (chloroform) | viscous liquid |
| 12 | triisopropanolamine | 1580 (chloroform) | viscous liquid |
| 13 | 3-dimethylamino-1-propylamine | 1575 (chloroform) 1575 (chloroform) | viscous liquid (1:1 salt) viscous liquid (1:2 salt) |

### (Example 4) Synthesis of salt (ionic liquid) of flurbiprofen and an organic amine compound

In the same manners as in Example 1 and Example 2, flurbiprofen salts shown in the following Table 4 were produced. In addition, disappearance of the absorption spectrum of carboxylic acid (starting material) from an infrared absorption spectrum and appearance of new absorption spectrum of a carboxyl ion are shown in Table 4.

**[Table 4]**

| sample No. | organic amine compound | flurbiprofen salt IR absorption (-COOH) CM⁻¹ | form/note |
|---|---|---|---|
| | none | 1700 (Nujol) | |
| 1 | eperisone | 1605 (neat) | viscous liquid |
| 2 | tolperisone | 1605 (neat) | viscous liquid |
| 3 | tramadol | 1600 (chloroform) | viscous liquid |
| 4 | dextromethorphan | 1605 (chloroform) | viscous liquid |
| 5 | diphenhydramine | 1600 (neat) | viscous liquid |
| 6 | dibucaine | 1595 (chloroform) | viscous liquid |
| 7 | lidocain | 1600 (neat) | viscous liquid |
| 8 | bupivacaine | 1580 (chloroform) | viscous liquid |
| 9 | donepezil | 1600 (chloroform) | viscous liquid |
| 10 | diethanolamine | 1570 (chloroform) | viscous liquid |
| 11 | triethanolamine | 1580 (Nujol) | crystal mp 93 - 94°C |
| 12 | diisopropanolamine | 1580 (chloroform) | viscous liquid |
| 13 | triisopropanolamine | 1580 (chloroform) | viscous liquid |
| 14 | 3-dimethylamino-1-propylamine | 1580 (chloroform) 1580 (chloroform) | viscous liquid (1:1 salt) viscous liquid (1:2 salt) |

### (Example 5) Synthesis of salt (ionic liquid) of etodolac and an organic amine compound

In the same manner as in Example 1 and Example 2, etodolac salts shown in the following Table 5 were produced. In addition, disappearance of the absorption spectrum of carboxylic acid (starting material) from an infrared absorption spectrum and appearance of new absorption spectrum of a carboxyl ion are shown in Table 5.

**[Table 5]**

| sample No. | organic amine compound | etodolac salt IR absorption (-COOH) cm¹ | form/note |
|---|---|---|---|
| | none | 1740 (Nujol) | |
| 1 | eperisone | 1605 (neat) | viscous liquid |
| 2 | tolperisone | 1605 (neat) | viscous liquid |
| 3 | tramadol | 1590 (Nujol) | crystal mp 136 - 137°C |
| 4 | dextromethorphan | 1600 (chloroform) | viscous liquid |
| 5 | diphenhydramine | 1590 (neat) | viscous liquid |
| 6 | dibucaine | 1600 (chloroform) | glue-like |
| 7 | lidocain | 1600 (chloroform) | viscous liquid |
| 8 | bupivacaine | 1585 (chloroform) | glue-like |
| 9 | donepezil | 1595 (chloroform) | glue-like |
| 10 | diethanolamine | 1575 (chloroform) | glue-like |
| 11 | triethanolamine | 1580 (Nujol) | crystal mp 94 - 95°C |
| 12 | diisopropanolamine | 1575 (chloroform) | viscous liquid |
| 13 | triisopropanolamine | 1575 (chloroform) | viscous liquid |
| 14 | 3-dimethylamino-1-propylamine | 1580 (chloroform) 1580 (chloroform) | viscous liquid (1:1 salt) glue-like (2:1 salt) |

### (Example 6) Synthesis of salt (ionic liquid) of loxoprofen and an organic amine compound

### (1) Synthesis of loxoprofen-tolperisone salt

a) When amount of equimolar tolperisone is used as an organic amine compound:
   Loxoprofen (740 mg, 3 mmol) and tolperisone (736 mg, 3 mmol) were dissolved in ethyl acetate (2 mL), and ethyl acetate was evaporated under reduced pressure to give a loxoprofen-tolperisone salt as a colorless starch syrup-like viscous liquid.
   infrared absorption spectrum (chloroform): 1735 cm-1 (carbonyl group in ring of loxoprofen), 1680 cm-1 (carbonyl group of tolperisone), 1605 cm-1 (-COO⁻, -COOH, chloroform, 1705 cm-1)
In the infrared absorption spectrum, the absorption spectrum (1715 cm-1) of carboxylic acid of loxoprofen (starting material) disappeared, and a new absorption spectrum (1605 cm-1) of carboxyl ion appeared.

b) When equimolar amount of tolperisone hydrochloride is used as an organic amine compound:
   A mixture of loxoprofen sodium salt (305 mg, 1 mmol) containing water (12%), tolperisone hydrochloride (282 mg, 1 mmol) and 2-propanol (5 mL) was stirred at room temperature for 30 min. The resulting precipitate was filtered off, and the filtrate was concentrated under reduced pressure to give a loxoprofen-tolperisone salt as a colorless starch syrup-like viscous liquid. This product showed an infrared absorption spectrum similar to that of the above-mentioned a).

### (2) Synthesis of other loxoprofen salts

In the same manner as in the above, equimolar salts (ionic liquid) of loxoprofen and an organic amine compound shown in Table 6 were produced. The absorption spectrum of carboxylic acid of loxoprofen (starting material) completely disappeared, and a new absorption spectrum of carboxyl ion shown in the following Table 6 appeared.

**[Table 6]**

| sample No. | organic amine compound | loxoprofen salt IR absorption (-COOH) CM⁻¹ | form/note |
|---|---|---|---|
| | none | 1715 (chloroform) | white crystalline powder |
| 1 | eperisone | 1605 (chloroform) | viscous liquid |
| 2 | tramadol | 1600 (neat) | viscous liquid |
| 3 | dextromethorphan | 1605 (chloroform) | viscous liquid |
| 4 | dibucaine | 1605 (neat) | viscous liquid |
| 5 | lidocain | 1600 (neat) | viscous liquid |
| 6 | bupivacaine | 1600 (chloroform) | viscous liquid partially crystallized during long-term preservation |
| 7 | diethanolamine | 1575 (neat) | viscous liquid |
| 8 | triethanolamine | 1575 (chloroform) | viscous liquid |
| 9 | diisopropanolamine | 1580 (neat) | viscous liquid |
| 10 | triisopropanolamine | 1580 (neat) | viscous liquid |

### (Example 7) Ointment containing ionic liquid of indomethacin-eperisone salt

The ionic liquid (0.5 g) of the indomethacin-eperisone salt obtained in Example 1 was measured, and glycerol (1.2 g), propylene glycol (0.2 g), t-butylhydroxytoluene (0.1 g), liquid paraffin (1 g), polybutene (0.1 g), hydrocarbon gel (2 g), aliphatic saturated hydrocarbon resin (4 g) and styrene-isoprene-styrene block copolymer (0.9 g) were mixed therewith to give a 5% ionic liquid ointment preparation.

### (Example 8) Plaster (tape) containing ionic liquid of indomethacin-eperisone salt

A plaster is produced from the indomethacin-eperisone salt (5 g) obtained in Example 1, diethyl sebacate (2 g), t-butylhydroxytoluene (1 g), 1-menthol (2 g), liquid paraffin (15 g), hydrocarbon gel (20 g), aliphatic saturated hydrocarbon resin (45 g) and styrene-isoprene-styrene block copolymer (10 g).

### (Example 9) Ointment containing equimolar ionic liquid of loxoprofen-eperisone

The loxoprofen-eperisone salt (ionic liquid 0.5 g) obtained in Example 6 was measured, and glycerol (1.2 g), propylene glycol (0.2 g), t-butylhydroxytoluene (0.1 g), liquid paraffin (1 g), polybutene (0.1 g), hydrocarbon gel (2 g), aliphatic saturated hydrocarbon resin (4 g) and styrene-isoprene-styrene block copolymer (0.9 g) were mixed therewith to give a 5% ionic liquid ointment preparation.

### (Example 10) Plaster (tape) containing equimolar ionic liquid of loxoprofen-eperisone

A plaster is produced from the equimolar salt of loxoprofen-eperisone (5 g) obtained in Example 6, diethyl sebacate (2 g), t-butylhydroxytoluene (1 g), l-menthol (2 g), liquid paraffin (15 g), hydrocarbon gel (20 g), aliphatic saturated hydrocarbon resin (45 g) and styrene-isoprene-styrene block copolymer (10 g).

### (Experimental Example 1) Solubility of ionic liquid of NSAID-eperisone salt

To show that the ionic liquids of NSAID-eperisone salts obtained in Examples 1-5 are dissolved well in solvents widely used for external preparations, the solubility is measured. First, the above-mentioned ionic liquid (1 g) is taken, various liposoluble solvents (10 g) are added thereto, and the mixtures are allowed to penetrate at room temperature. The liquids are stood still and, when undissolved ionic liquid is found, the supernatant is collected and the concentration of NSAID is measured by liquid chromatography, based on which the solubility in the solvent used is calculated.

### (Experimental Example 2) Evaluation test of skin permeability of ionic liquid of NSAID-organic amine salt

As a receptor solution, saline is placed in an upright Franz-type diffusion cell, and an isolated abdominal skin of Wistar rat is applied thereto. A solution of NSAID organic amine salt in a liposoluble solvent is applied to the rat skin. The concentration of NSAID in the receptor solution is measured at 32°C over time, and the amount of NSAID that permeated the skin is measured. As a comparison control, NSAID itself is dissolved in a liposoluble solvent, and the amount of NSAID that permeated the rat skin is measured in the same manner.

### (Experimental Example 3) Solubility of ionic liquid of equimolar salt of NSAID and eperisone

To show that the ionic liquid of an equimolar salt of loxoprofen and an organic amine compound obtained in Example 6 is dissolved well in a solvent widely used for external preparations, the solubility is measured. First, the above-mentioned ionic liquid (1 g) is taken, various liposoluble solvents (10 g) are added thereto, and the mixtures are allowed to penetrate at room temperature. The liquids are stood still and, when undissolved ionic liquid is found, the supernatant is collected and the concentration of loxoprofen is measured by liquid chromatography, based on which the solubility in the solvent used is calculated.

### (Experimental Example 4) Evaluation test of skin permeability of ionic liquid of equimolar salt of oxoprofen and organic amine compound

As a receptor solution, saline is placed in an upright Franz-type diffusion cell, and an isolated abdominal skin of Wistar rat is applied thereto. A solution of an equimolar salt of loxoprofen and organic amine compound in a liposoluble solvent is applied to the rat skin. The concentration of loxoprofen in the receptor solution is measured at 32°C over time, and the amount of loxoprofen that permeated the skin is measured. As a comparison control, loxoprofen itself is dissolved in a liposoluble solvent, and the amount of loxoprofen that permeated the rat skin is measured in the same manner.

### (Experimental Example 5) Solubility of ionic liquid of NSAID-eperisone salt

The solubility of an eperisone salt of NSAID, and a free acid of NSAID in diethyl sebacate widely used for external preparations was measured.
First, each ionic liquid corresponding to 5 g of NSAID was taken, diethyl sebacate (50 g) was added thereto, and the mixture was shaken at room temperature for 30 min. In the same manner, a free acid (5 g) of each NSAID was taken, diethyl sebacate (50 g) was added thereto, and the mixture was shaken at room temperature for 30 min. The solutions were stood still and, when an undissolved matter was found, diethyl sebacate was further added, and the mixture was shaken. As a result, the ionic liquid of NSAID and eperisone was completely dissolved in diethyl sebacate (50 g), but each free acids of indomethacin, diclofenac and loxoprofen alone were not dissolved.

As an index showing the solubility of NSAID free acid and NSAID-eperisone salt in diethyl sebacate, the amount (g) of diethyl sebacate necessary for dissolving NSAID (1 g) or an eperisone salt corresponding to NSAID (1 g) is shown in Table 7.

**[Table 7]**

| | NSAID | NSAID-eperisone salt |
|---|---|---|
| indomethacin | not less than 100 g | not more than 10 g |
| diclofenac | not less than 100 g | not more than 10 g |
| loxoprofen | not less than 100 g | not more than 10 g |
| ketoprofen | not more than 10 g | not more than 10 g |
| flurbiprofen | not more than 10 g | not more than 10 g |
| etodolac | not more than 10 g | not more than 10 g |

### (Experimental Example 6) Skin permeability test of NSAID-organic amine salt using isolated rat abdominal skin

### <Preparation of solution preparation for test>

An equimolar salt of NSAID and organic amine was dissolved in a mixture of diethyl sebacate and propylene glycol to give a solution preparation. For comparison, a solution preparation (control) of NSAID was prepared in the same manner.

### <Rat skin permeation test>

As a receptor solution, saline-ethanol solution (9:1, 8 ml) was placed in an upright Franz-type diffusion cell (effective diffusion area: 1 cm²), and the solution was stirred and maintained at 32°C. An isolated abdominal shaved skin of 6-week-old Wistar rat was applied to the Franz cell, and the prepared solution preparation (0.15 ml) was applied to the skin. A part (300 µL) of the receptor solution was taken at predetermined time intervals, the drug concentration in the receptor solution was measured by high performance liquid chromatography, and the drug permeation amount per 1 cm² rat skin was calculated. The results are shown in Tables 8-13. All of the NSAID-organic amine salts showed higher skin permeability than the control NSAID-solution preparation.

Unless otherwise indicated, in the following Experimental Examples, the drug concentration of the solution preparation is 2.5 wt% as NSAID free acid, and the skin permeation amount is an accumulated amount (µg/cm²) up to 6 hours.

**[Table 8]**

| rat skin permeation amount of indomethacin-organic amine salt | | |
|---|---|---|
| | compound | indomethacin permeation amount µg/cm² |
| control | indomethacin | 6.9 |
| | indomethacin-tolperisone salt | 14.7 |
| | indomethacin-tramadol salt | 9.8 |
| | indomethacin-TIPA salt | 15.8 |

| | | |
|---|---|---|
| (TIPA: triisopropanolamine) | | |

**[Table 9]**

| rat skin permeation amount of diclofenac-organic amine salt | | |
|---|---|---|
| | compound | diclofenac permeation amount µg/cm² |
| control | diclofenac | 8.8 |
| | diclofenac-donepezil salt | 14.3 |
| | diclofenac-tolperisone salt | 20.3 |

**[Table 10]**

| rat skin permeation amount of ketoprofen-organic amine salt | | |
|---|---|---|
| | compound | Ketoprofen permeation amount µg/cm² |
| control | ketoprofen | 36.1 |
| | ketoprofen-tolperisone salt | 60.3 |
| | ketoprofen-lidocain salt | 70.2 |

**[Table 11]**

| rat skin permeation amount of flurbiprofen-organic amine salt | | |
|---|---|---|
| | compound | flurbiprofen permeation amount µg/cm² |
| control | flurbiprofen | 20.2 |
| | flurbiprofen-lidocain salt | 91.0 |
| | flurbiprofen-tolperisone salt | 46.3 |
| | flurbiprofen-tramadol salt | 78.1 |
| | flurbiprofen-donepezil salt | 28.3 |
| | flurbiprofen-TIPA salt | 91.8 |

| | | |
|---|---|---|
| (TIPA: triisopropanolamine) | | |

**[Table 12]**

| rat skin permeation amount of etodolac-organic amine salt | | |
|---|---|---|
| | compound | etodolac permeation amount µg/cm² |
| control | etodolac | 10.4 |
| | etodolac-tolperisone salt | 22.3 |
| | etodolac-eperisone salt | 20.5 |

**[Table 13]**

| rat skin permeation amount of loxoprofen-organic amine salt | | |
|---|---|---|
| | compound | loxoprofen permeation amount µg/cm² |
| control | loxoprofen sodium salt | 27.1 |
| control | loxoprofen free acid | 49.5 |
| | loxoprofen-tolperisone salt | 82.3 |
| | loxoprofen-eperisone salt | 91.2 |
| | loxoprofen-lidocain salt | 103.4 |
| | loxoprofen-DIPA salt | 68.6 |

| | | |
|---|---|---|
| (DIPA: diisopropanolamine) | | |

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the claims.

This application is based on patent application Nos. JP 2007-18005, JP 2007-31188 and JP 2007-69620 filed in Japan, the contents of which are incorporated in full herein by this reference.

## Claims

1. A 1:1 salt comprising a carboxylic acid non-steroidal anti-inflammatory drug below and an organic amine compound,
a) salts of indomethacin:
dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
b) salts of ketoprofen:
lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
c) salts of flurbiprofen:
lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
d) salts of diclofenac:
dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
e) salts of etodolac:
lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
f) salts of loxoprofen:
lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt.

2. An ionic liquid which is a 1:1 salt comprising a carboxylic acid non-steroidal anti-inflammatory drug below and an organic amine compound, and is a liquid at ambient temperature (25°C),
a) salts of indomethacin:
dibucaine salt, bupivacaine salt, diphenhydramine salt, tramadol salt, eperisone salt, tolperisone salt, dextromethorphan salt, donepezil salt, triethanolamine salt, triisopropanolamine salt, 3-dimethylamino-1-propylamine salt,
b) salts of ketoprofen:
lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt,
c) salts of flurbiprofen:
lidocain salt, dibucaine salt, bupivacaine salt, diphenhydramine salt, tramadol salt, eperisone salt, tolperisone salt, dextromethorphan salt, donepezil salt, diethanolamine salt, diisopropanolamine salt, triisopropanolamine salt, 3-dimethylamino-1-propylamine salt,
d) salts of diclofenac:
lidocain salt, bupivacaine salt, diphenhydramine salt, eperisone salt, tolperisone salt, dextromethorphan salt, donepezil salt,
e) salts of etodolac:
lidocain salt, dibucaine salt, bupivacaine salt, diphenhydramine salt, eperisone salt, tolperisone salt, dextromethorphan salt, diethanolamine salt, diisopropanolamine salt, triisopropanolamine salt, donepezil salt, 3-dimethylamino-1-propylamine salt,
f) salts of loxoprofen:
lidocain salt, dibucaine salt, bupivacaine salt, diphenhydramine salt, tramadol salt, eperisone salt, tolperisone salt, dextromethorphan salt, donepezil salt, diethanolamine salt, triethanolamine salt, diisopropanolamine salt, triisopropanolamine salt, 3-dimethylamino-1-propylamine salt.

3. An external preparation comprising the ionic liquid of claim 2.

4. A method of converting a carboxylic acid non-steroidal anti-inflammatory drug to an ionic liquid, which comprises adding an organic amine compound to a carboxylic acid non-steroidal anti-inflammatory drug, wherein the organic amine compound is at least one kind selected from the group consisting of lidocain, dibucaine, bupivacaine, diphenhydramine, tramadol, eperisone, tolperisone, dextromethorphan, donepezil, diethanolamine, triethanolamine, diisopropanolamine, triisopropanolamine and 3-dimethylamino-1-propylamine.

5. The method of claim 4, wherein the carboxylic acid non-steroidal anti-inflammatory drug and the organic amine compound form an equimolar salt.

6. The method of claim 4 or 5, wherein the carboxylic acid non-steroidal anti-inflammatory drug is selected from the group consisting of indomethacin, ketoprofen, flurbiprofen, etodolac and loxoprofen.

7. The method of any one of claims 4 to 6, wherein the carboxylic acid non-steroidal anti-inflammatory drug is loxoprofen.
